(19) Europäisches Patentamt
European Patent Office
Office européen des brevets

(11) Veröffentlichungsnummer: **0 463 556 A2**

(12) **EUROPÄISCHE PATENTANMELDUNG**

(21) Anmeldenummer: **91110054.3**

(51) Int. Cl.5: **C07C 63/72**, C07C 51/265

(22) Anmeldetag: **19.06.91**

(30) Priorität: **25.06.90 DE 4020186**

(43) Veröffentlichungstag der Anmeldung:
**02.01.92 Patentblatt 92/01**

(84) Benannte Vertragsstaaten:
**CH DE FR GB IT LI**

(71) Anmelder: **HOECHST AKTIENGESELLSCHAFT**
**Postfach 80 03 20**
**W-6230 Frankfurt am Main 80(DE)**

(72) Erfinder: **Röhrscheid, Freimund**
**Amselweg 24**
**W-6233 Kelkheim(DE)**
Erfinder: **Appel, Wolfgang**
**Taunusstrasse 74A**
**W-6233 Kelkheim(Taunus)(DE)**
Erfinder: **Siegemund, Günter**
**Frankfurter Strasse 21**
**W-6238 Hofheim(DE)**

(54) **Teilfluorierte Tetracarbonsäure sowie deren Dianhydrid, Verfahren zu ihrer Herstellung und ihre Verwendung.**

(57) Verbindung der Formel

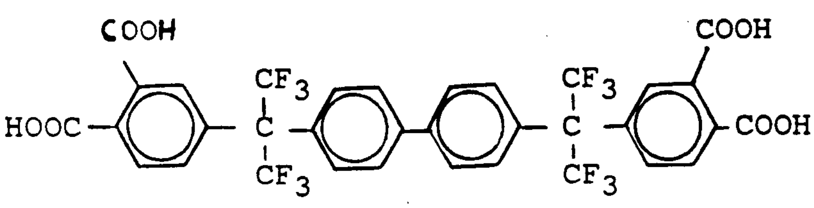

(I)

sowie deren Dianhydrid werden hergestellt durch Luftoxidation in Gegenwart eines Katalysatorgemisches aus mindestens 2 Schwermetallsalzen sowie Brom in einem sauren organischen Medium. Die Verbindungen können zur Herstellung von teilfluorierten Polykondensaten wie Polyimiden, Polycarbonsäureimiden, Polyamidcarbonsäureestern, Polyamiden und Imidoligomeren eingesetzt werden.

EP 0 463 556 A2

Die Erfindung betrifft eine teilfluorierte Tetracarbonsäure, im speziellen das 4,4'-Bis[2-(3,4-dicarboxyphenyl)hexafluorisopropyl]biphenyl, dessen Dianhydrid, Verfahren zu ihrer Herstellung sowie ihre Verwendung.

Die Herstellung des 4,4'-Bis [2-(3,4-dicarboxyphenyl)hexafluorisopropyl]diphenylethers durch Luftoxidation in saurem Medium in Gegenwart eines Katalysatorgemisches ist bekannt [DE-A-37 39 796].

Gegenstand der Erfindung ist eine Verbindung der Formel

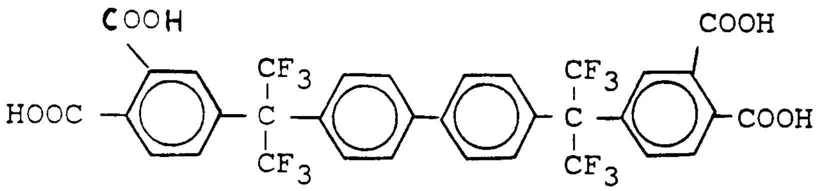

(I)

sowie deren Dianhydrid, Verfahren zu ihrer Herstellung und ihre Verwendung als Monomerkomponente für Polykondensate, insbesondere Polyimide.

Die Herstellung der Verbindung gemäß der Erfindung erfolgt durch Oxidation des 4,4'-Bis[2-(3,4-dialkylphenyl)hexafluorisopropyl]biphenyls mit molekularem Sauerstoff in einem sauren organischen Medium, wobei das saure Medium zu mindestens 40 Gew.-% aus einer Monocarbonsäure mit 1 bis 4 Kohlenstoffatomen, insbesondere Essigsäure oder Propionsäure oder deren Mischungen, besteht, in Gegenwart einer Katalysatorkombination aus mindestens 2 Schwermetallsalzen, insbesondere des Kobalts und Mangans, sowie Brom. Daneben können noch Cerionen vorhanden sein. Essigsäure ist wegen ihrer größeren Beständigkeit gegen einen oxidativen Abbau vorzuziehen. Das Verhältnis von saurem Medium zu der zu oxidierenden Biphenylverbindung maximal von 40:60 Gew.-%, bezogen auf die Gesamtreaktionsmasse, betragen.

Die eingesetzte Biphenylverbindung

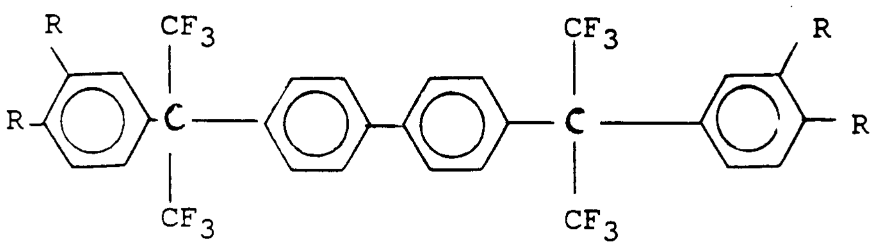

(II),

in der R gleich niederes Alkyl mit 1-4 C-Atomen ist, wobei Alkyl vorzugsweise Methyl, Ethyl und Isopropyl, insbesondere Methyl ist, wird im allgemeinen nach drei verschiedenen Methoden hergestellt, und zwar:

a) durch Kondensation von einem Mol eines Dicarbinols der Formel

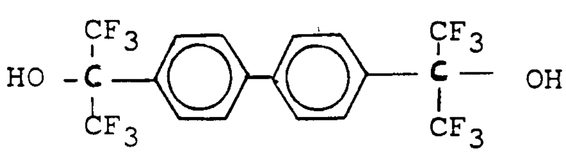

(III)

mit mindestens 2 Mol einer Verbindung mit der Formel

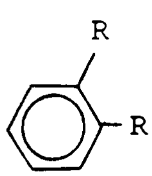

(IV),

wobei R die vorgenannte Bedeutung hat,
oder
b) durch Kondensation von mindestens 2 Mol einer Verbindung der Formel

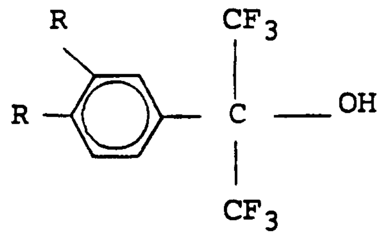

(V),

wobei R die vorgenannte Bedeutung hat, mit einem Mol Biphenyl (VI), jeweils in Gegenwart von Fluorwasserstoff
oder
c) durch Bildung der Kohlenstoff-Kohlenstoff-Bindung zwischen 2 gleichen teilfluorierten aromatischen Verbindungen der Formel

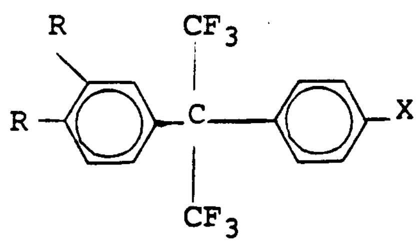

(VII),

wobei R die vorgenannte Bedeutung hat, nach einem literaturbekannten Verfahren, das sich zur Bildung von Aryl-Arylbindungen eignet, z. B. J. Org. Chem. 51, 2627 (1986). X ist Halogen, vorzugsweise Chlor.

Verbindungen der Formel (III), die beim Vorgehen nach a) eingesetzt werden, sind in der US-Patentschrift 3,355,500 und in J. Org. Chem. 30, 998 (1965) beschrieben. Verbindungen der Formel (V), die nach Methode b) zu den Verbindungen gemäß Formel (II) umgesetzt werden, sind ebenfalls in J. Org. Chemie 30, 998-1001 (1965) beschrieben.

Die Reaktion nach Methode a) und b) wird bei einer Temperatur von 80 bis 180 °C, vorzugsweise von 100 bis 160 °C, durchgeführt.

Für die Reaktion nach Methode a) und b) ist ein Zeitraum von 20 bis 90 Stunden, vorzugsweise von 40 bis 70 Stunden, erforderlich.

Das Molverhältnis der eingesetzten Reaktionspartner wird bestimmt bei Methode a) durch das Verhältnis der Verbindung (III) zu Verbindung (IV), sowie bei Methode b) durch das Verhältnis des Biphenyls zu Verbindung (V); es beträgt jeweils mindestens 1:2, vorzugsweise 1:2,2 bis 1:4,4.

Der Anteil an Fluorwasserstoff, der bei der Reaktion zur Herstellung der Verbindungen gemäß Formel (II) notwendig ist, wird bei Methode a) auf die Verbindung (III) bezogen und liegt im allgemeinen bei einem Molverhältnis von 1:7 bis 1:25, vorzugsweise 1:8 bis 1:12. Bei der Methode b) beträgt das Molverhältnis der Verbindung (V) zu Fluorwasserstoff im allgemeinen 1:6 bis 1:15, vorzugsweise 1:8 bis 1:12.

Zur Aufarbeitung des Reaktionsgutes wird im allgemeinen der Fluorwasserstoff nach Beendigung der Reaktion bei ca. 80 °C aus dem Reaktor abgegast und der verbleibende Rückstand, gegebenenfalls nach Verdünnen mit einem organischen Lösungsmittel, dem Reaktor entnommen, vorzugsweise bei einer Temperatur von 20 bis 30 °C.

Als Lösungsmittel, die dabei verwendet werden können, eignen sich aliphatische Kohlenwasserstoffe mit 5 bis 10 C-Atomen, aromatische Kohlenwasserstoffe mit 6 bis 8 C-Atomen und ein- oder mehrfach chlorierte aliphatische Kohlenwasserstoffe mit 1 bis 4 C-Atomen im Alkylrest. Beispiele hierfür sind n-Hexan, n-Heptan, Toluol, die verschiedenen Xylole, sowie Di- und Trichlormethan, vorzugsweise Toluol, Di- oder Trichlormethan.

Das erhaltene Rohgemisch wird mit Wasser versetzt, gewaschen und abgetrennt. Im allgemeinen fallen die gereinigten Produkte als farblose Kristallisate an.

Zur weiteren Reinigung kann das Reaktionsgut einer Umkristallisation aus einem organischen Lösungsmittel unterworfen werden, oder es erfolgt ein Ausrühren in organischen Lösungsmitteln, vorzugsweise in Isopropanol, Methanol oder 1-Chlorpropan.

Die Herstellung der Verbindung gemäß Formel (VII) ist nach bekannten Verfahren möglich aus Verbindungen der Formel (V) und Arylhalogeniden.

Die Bildung der Aryl-Arylbindung zwischen zwei Komponenten nach Formel (VII) wird durchgeführt in einem polaren aprotischen Lösungsmittel wie Dimethylacetamid oder Dimethylformamid in Gegenwart eines Gemisches aus 1 bis 10 Mol%, vorzugsweise 3 bis 6 Mol%, eines Nickel-(II)-Salzes, vorzugsweise $NiCl_2$ oder $NiBr_2$, und 5 bis 40 Mol%, vorzugsweise 20 bis 30 Mol%, einer organischen Phosphor-(III)-Verbindung, vorzugsweise Triphenylphosphin, und Zinkpulver in einer Menge von 120 bis 160 Mol%, bezogen auf das eingesetzte Arylhalogenid.

Die Reaktion wird in einer Inertgasatmosphäre, insbesondere von Stickstoff oder Argon, bei einer Temperatur von 40 bis 80 °C ausgeführt; die Reaktion dauert 2 bis 8 Stunden.

Der Feststoffanteil wird abfiltriert und das Filtrat nach Zusatz eines nicht mit Wasser mischbaren Lösungsmittels, z.B. ein ein- oder mehrfach chlorierter aliphatischer Kohlenwasserstoff mit 1-4 Kohlenstoffatomen im Alkylrest, insbesondere Di- oder Trichlormethan, Essigsäureester oder Diethylether, mehrfach mit Wasser gewaschen. Dabei stellt sich eine Phasentrennung ein. Nach Trocknung der organischen Phase wird das Lösungsmittel abdestilliert und das zurückbleibende Produkt durch Umkristallisation gereinigt.

Für den vollständigen Ablauf der Oxidation sind Bromidionen unbedingt notwendig. Die beiden Schwermetallsalze, insbesondere die des Kobalts und des Mangans, werden im allgemeinen im Verhältnis von 3:1 bis 1:3, vorzugsweise 1:1 eingesetzt. Die Summe der Konzentrationen der beiden Kationen beträgt im allgemeinen 0,01 bis 0,2, vorzugsweise 0,02 bis 0,12 und insbesondere 0,04 bis 0,08 g/Atom/kg Gesamtmasse. Das Verhältnis der Summe der Metallsalze, vorzugsweise der von Kobalt und Mangan, zu Brom ist im allgemeinen 1:0,01 bis 1:0,8, vorzugsweise 1:0,05 bis 1:0,4.

Es ist auch möglich, zusätzlich zu den beiden Metallionen des Katalysators auch Cerionen einzusetzen. Diese katalysieren die Oxidation unvollständig oxidierter Zwischenstufen. Ihre Anwesenheit erhöht die Reinheit und die Ausbeute der teilfluorierten Tetracarbonsäure. Die Cerionen werden dem Katalysator im Verhältnis der Summe der Kobalt- und Manganionen zu Cerionen wie 1:0,02 bis 1:1,2, vorzugsweise 1:0,05 bis 1:0,6, zugefügt.

Wird ein Gemisch der Metallionen von Kobalt und Cer verwendet, so beträgt das Molverhältnis der beiden Metalle im allgemeinen 1:0,02 bis 1:1,2, wobei das Verhältnis der Metalle zu Brom wie vorstehend beschrieben ist. Die Molverhältnisse beziehen sich stets auf die Gesamtmasse, d.h. auf die Summe der zu oxidierenden Verbindung, Lösungsmittel und Katalysator. Vorzugsweise werden als Metallsalze die entsprechenden Acetate eingesetzt.

Brom kann in Form von Bromiden, z.B. der Bromide der Alkalimetalle einschließlich des Ammoniumbromids und der Metalle Kobalt, Mangan und Cer, oder als Lösung von Bromwasserstoff in Wasser oder Eisessig eingesetzt werden. Es können auch bromhaltige organische Verbindungen verwendet werden, die während der Oxidation zerfallen und Bromionen freisetzen, z.B. Tetrabrommethan.

Die Oxidation wird im allgemeinen bei einer Temperatur von 120 bis 220, vorzugsweise 140 bis 190 und insbesondere 155 bis 180 °C durchgeführt. Der Druck im Reaktor beträgt im allgemeinen 5 bis 40, vorzugsweise 10 bis 30 und insbesondere 14 bis 20 bar.

Für die Verfahrensweise ist es günstig, daß die zur Oxidation benötigte Luft nahe dem Boden des Reaktors in die Flüssigphase eingeleitet und vorzugsweise durch starkes Rühren oder durch spezielle Düsen in der Flüssigphase fein verteilt wird. Besonders günstig ist es, ein Oxidationsgemisch zu verwenden, dessen Sauerstoffgehalt durch Beimischen von reinem Sauerstoff auf einen Anteil von über 21 Volumen-% erhöht wurde. Durch diese Maßnahme werden hohe Sauerstoff-Partialdrucke in den in die Flüssigphase eintretenden Gasblasen erreicht. Es ist vorteilhaft, wenn der Sauerstoff-Partialdruck an der Austrittsstelle der Einleitungsvorrichtung mindestens 1 bar, vorzugsweise 2 bis 15 und insbesondere 3 bis 10 bar, beträgt.

Es ist für die Verfahrensführung weiterhin günstig, daß der Restsauerstoffgehalt des Abgases bestimmte Werte nicht unterschreitet. Der Sauerstoff-Partialdruck wird definiert durch die Formel

$$P_{O_2} = \text{Volumen-\%}\ O_2 \cdot (P_{ges} - P_{ac})$$

d.h. er ist das mathematische Produkt aus dem Restsauerstoffgehalt und der Differenz aus dem Gesamtdruck und dem Essigsäuredampfdruck bei der vorliegenden Reaktionstemperatur. Dieser Sauerstoff-Partialdruck in der Gasphase über der Reaktionslösung soll 0,2 bar nicht unterschreiten und beträgt bevorzugt 0,35 bis 2,8, insbesondere 0,45 bis 1,3 bar.

Nach Beendigung der stark exothermen Reaktion ist es zweckmäßig, zur Vervollständigung der Oxidation aller Alkylgruppen den Reaktor für 1 bis 3 Stunden, vorzugsweise für etwa 2 Stunden, auf 150 bis 190 °C, vorzugsweise 160 bis 180 °C, bei einem Sauerstoffpartialdruck von 0,4 bis 2 bar, vorzugsweise 0,5 bis 1,3 bar, zu halten.

Einen wesentlichen Einfluß auf die Durchführung des erfindungsgemäßen Verfahrens hat die Wasserkonzentration des sauren Mediums, in dem die Reaktion durchgeführt wird.

Zwar können die Tetraalkylverbindungen auch in - beispielsweise - Essigsäure mit einer Wasserkonzentration von 15 Gew.% und mehr oxidiert werden, jedoch vermindert sich dabei Ausbeute und vor allem die Reinheit der erhaltenen Produkte und die Oxidation aller vier Alkylgruppen läuft nur noch unvollständig ab. Andererseits wurde festgestellt, daß in wasserfreier Essigsäure die Metallionen des Katalysators durch Tetracarbonsäuren ausgefällt und damit inaktiviert werden. Der Bereich für die Wasserkonzentration, in dem die Metallionen gelöst bleiben und in dem die Oxidation vollständig abläuft, beträgt 2 bis 12, vorzugsweise 2 bis 7 und insbesondere 3 bis 5 Gew.% Wasser in der Monocarbonsäure.

Zur Überführung in das Dianhydrid wird die nach dem erfindungsgemäßen Verfahren erhaltene Tetracarbonsäure in üblicher Weise mit Acetanhydrid behandelt und nach bekannten Methoden aus der Reaktionslösung gewonnen.

Die erfindungsgemäßen Verbindungen werden vor allem zur Herstellung von Polykondensaten wie Polyimiden, Polycarbonsäureamiden, Polyamidcarbonsäureestern, Polyamiden und Imidoligomeren eingesetzt, die u.a. niedrige Schmelzpunkte, hohe Löslichkeit, niedrige Dielektrizitätskonstanten, erhöhte thermische Stabilität aufweisen.

## Beispiele

### 1) a) 4,4'-Bis[2-(3,4-dicarboxyphenyl)hexafluorisopropyl]biphenyl (12F-Biphenyl-tetracarbonsäure)

165,6 g 4,4'-Bis[2-(3,4-dimethylphenyl)hexafluorisopropyl]biphenyl, 2,49 g $Co(OAc)_2.4H_2O$, 2,45 g $Mn(OAc)_2.4H_2O$, 0,45 g HBr entsprechend 4,5 g 10%iger HBr-Lösung in Eisessig und 450 g Eisessig wurden in einem Ein-Liter-Autoklaven, der mit Rührer, Heizmantel, Gaseinleitungsrohr, Thermometer, Rückflußkühler und einem Sauerstoffmeßgerät in der Abgasleitung ausgerüstet war, vorgelegt. Unter 16 bar Stickstoff wurde das Reaktionsgemisch auf 150 °C erhitzt. Dann wurde durch das nah am Boden befindliche Einleitungsrohr Luft eingeleitet. Die exotherme Reaktion setzte sofort unter Sauerstoffaufnahme ein, wobei die Temperatur bis auf 185 °C stieg. Es wurde soviel Luft eingeleitet, daß der Sauerstoffgehalt im Abgas zwischen 5 und 9 Vol-% lag.

Die exotherme Reaktion dauerte 1 Stunde. Anschließend wurde die Luft durch eine Mischung von Stickstoff-Sauerstoff (9:1) ersetzt und die Temperatur durch Heizen noch 45 Minuten bei 175 °C gehalten.

Die auf 100 °C abgekühlte pastöse Reaktionsmischung wurde dem Reaktionsgefäß entnommen, unter Rühren auf 20 °C abgekühlt und abgesaugt. Der voluminöse Filterkuchen wurde viermal mit jeweils 150 g Eisessig gewaschen. Der Filterkuchen (530 g) (Tetracarbonsäure : Essigsäure ca. 1:2) wurde bei 70 °C/65 mbar im Luftstrom getrocknet.

| | |
|---|---|
| Ausbeute: | 176,3 g (93,9 % d.Th.) |
| Festpunkt: | 208-212 °C (Dehydratisierung) bei 270 °C vollständig geschmolzen |
| Carboxylgruppengehalt: | 5,13 mVal COOH/g (ber. 5,11) |
| Farbe: | beige |

| Analyse für $C_{34}H_{18}F_{12}O_8$: | | | |
|---|---|---|---|
| ber.: | C 52,19 % | H 2,32 % | F 29,14 % |
| gef.: | C 52,10 % | H 2,25 % | F 29,30 % |

### 2) 12F-Biphenyl-tetracarbonsäuredianhydrid

Der voluminöse essigsäurefeuchte Filterkuchen (530 g) aus der Isolierung als 12F-Biphenyl-tetracarbonsäure wurde zusammen mit 200 g Eisessig in einen Zwei-Liter-Vierhalskolben gefüllt. Unter Rühren wurde bei etwa 85 °C im Laufe von 30 Minuten 102 g (1,0 Mol) Acetanhydrid eingetropft. Die Temperatur stieg auf 120 °C, und die dünnflüssige Suspension wurde für eine Stunde bei diesem Wert gehalten (6 Gew.%

Acetanhydrid in dem Eisessig).

Die Reaktionslösung wurde unter Rühren auf 20 °C abgekühlt und der Feststoffanteil abgesaugt. Der Filterkuchen wurde sechsmal mit je 60 g einer Mischung aus 92 Gew.-% Eisessig und 8 Gew.-% Acetanhydrid gewaschen, abgesaugt und bei 100 °C/65 mbar im Luftstrom getrocknet.

Ausbeute: 147,3 g 12F-Biphenyl-tetracarbonsäuredianhydrid (82,2 % d.Th.)
Festpunkt: 276-278 °C

Anhydridgruppengehalt bestimmt durch Titration mit $^{n}/10$ Natronlauge/$^{n}/10$ Salzsäure:
2,66 mVal Anhydrid/g (ber. 2,68)

| Analyse für $C_{34}H_{14}F_{12}O_6$: | | | |
|---|---|---|---|
| ber.: | C 54,70 % | H 1,89 % | F 30,54 % |
| gef.: | C 54,6 % | H 2,0 % | F 30,7 % |

**Patentansprüche**

**1.** Verbindung der Formel

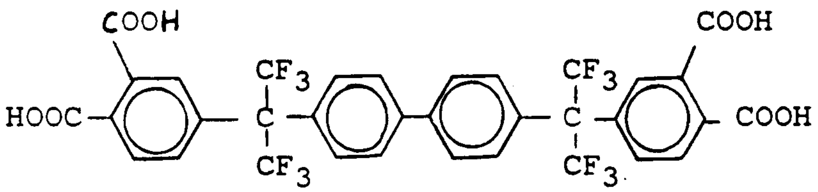

(I)

sowie deren Dianhydrid.

**2.** Verfahren zur Herstellung einer Verbindung der Formel

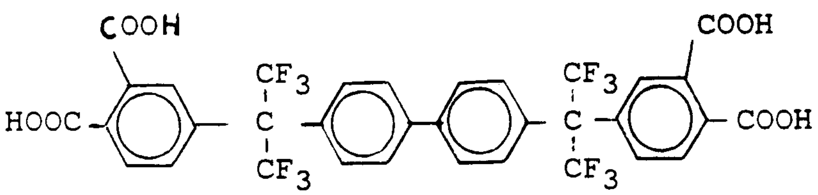

(I)

oder deren Anhydrid durch Luftoxidation in saurem Medium bei erhöhtem Druck und erhöhter Temperatur in Gegenwart eines Katalysatorgemisches, dadurch gekenzeichnet, daß 4,4'-Bis[2-(3,4-dialkylphenyl)hexafluorisopropyl]biphenyl durch Einleiten von Luftsauerstoff in ein saures organisches Medium, welches aus einer Monocarbonsäure mit 1 bis 4 Kohlenstoffatomen besteht, bei einer Temperatur von 120 bis 220 °C und einem Druck von 5 bis 40 bar in Gegenwart von mindestens 2 Schwermetallsalzen sowie von Bromidionen oxidiert und als Produkt nach Formel I isoliert wird oder dieses unter Verwendung von Acetanhydrid in das Dianhydrid der Verbindung der Formel (I) überführt wird.

**3.** Verfahren nach Anspruch 2, dadurch gekennzeichnet, daß das saure organische Medium zu mindestens 40 Gew.-% aus Essigsäure und/oder Propionsäure besteht, bezogen auf das Gesamtgewicht.

**4.** Verfahren nach Anspruch 2 oder 3, dadurch gekennzeichnet, daß die Reaktionstemperatur 140 bis 190, insbesondere 155 bis 180 °C beträgt.

**5.** Verfahren nach einem oder mehreren der Ansprüche 2 bis 4, dadurch gekennzeichnet, daß die Oxidation bei einem Druck von 10 bis 30, insbesondere von 14 bis 20 bar durchgeführt wird.

6. Verfahren nach einem oder mehreren der Ansprüche 2 bis 5, dadurch gekennzeichnet, daß der zur Oxidation eingesetzte Luftsauerstoff ein Sauerstoffgehalt von über 21 Vol-% besitzt und daß an der Einleitstelle des Sauerstoffs der Sauerstoffpartialdruck mindestens 1 bar, vorzugsweise 2 bis 15, insbesondere 3 bis 10 bar beträgt.

7. Verfahren nach einem oder mehreren der Ansprüche 2 bis 6, dadurch gekennzeichnet, daß der Sauerstoffpartialdruck in der Gasphase über dem Reaktionsmedium mindestens 0,2 bar, vorzugsweise 0,35 bis 2,8, insbesondere 0,45 bis 1,3 bar beträgt.

8. Verfahren nach einem oder mehreren der Ansprüche 2 bis 7, dadurch gekennzeichnet, daß Schwermetallsalze von Kobalt, Mangan und/oder Cer eingesetzt und diese als Acetate verwendet werden.

9. Verfahren nach einem oder mehreren der Ansprüche 2 bis 8, dadurch gekennzeichnet, daß Brom in Form von Bromiden oder als Lösung von Bromwasserstoff in Wasser oder Eisessig eingesetzt wird.

10. Verfahren nach einem oder mehreren der Ansprüche 2 bis 9, dadurch gekennzeichnet, daß das Molverhältnis von Kobalt zu Mangan gleich 3:1 bis 1:3 ist, wobei die Summe der Konzentration der beiden Elemente Kobalt und Mangan gleich 0,01 bis 0,20 g-Atom/kg Gesamtreaktionsmasse, vorzugsweise 0,02 bis 0,12, insbesondere 0,04 bis 0,08 g-Atom/kg beträgt.

11. Verfahren nach einem oder mehreren der Ansprüche 2 bis 10, dadurch gekennzeichnet, daß das Molverhältnis der Summe von Kobalt und Mangan zu Brom 1:0,01 bis 1:0,8, vorzugsweise 1:0,05 bis 1:0,4 beträgt.

12. Verfahren nach einem oder mehreren der Ansprüche 2 bis 11, dadurch gekennzeichnet, daß als zusätzliches Metallion Cer im Katalysator enthalten ist, wobei das Molverhältnis der Summe von Kobalt und Mangan zu Cer 1:0,02 bis 1:1,2, vorzugsweise 1:0,05 bis 1:0,6 beträgt.

13. Verfahren nach einem oder mehreren der Ansprüche 2 bis 12, dadurch gekennzeichnet, daß das Molverhältnis von Kobalt zu Cer 1:0,02 bis 1:1,2 beträgt.

14. Verfahren nach einem oder mehreren der Ansprüche 2 bis 13, dadurch gekennzeichnet, daß der Reaktionsansatz nach Beendigung der exothermen Reaktion für 1 bis 3 Stunden auf 150 bis 190 °C bei einem Sauerstoffpartialdruck von 0,4 bis 2 bar gehalten wird.

15. Verfahren nach einem oder mehreren der Ansprüche 2 bis 14, dadurch gekennzeichnet, daß die Reaktion bei einer Wasserkonzentration der Monocarbonsäure von 2 bis 12, vorzugsweise 2 bis 7 und insbesondere bei 3 bis 5 Gew.% durchgeführt wird.

16. Verwendung der Verbindungen gemäß Anspruch 1 zur Herstellung von Polykondensaten.

17. Verwendung nach Anspruch 16, dadurch gekennzeichnet, daß Polyimide, Polycarbonsäureamide, Polyamidcarbonsäureester, Polyamide und Imidoligomere hergestellt werden.